# EUROPEAN PATENT APPLICATION

(11) **EP 3 130 884 A1**
(43) Date of publication of application: **15.02.2017**
(21) Application number: 15181026.4
(22) Date of filing: 14.08.2015
(51) Int. Cl.: G01B 5/28, G01N 19/08, G01N 33/34, G01N 3/56

(54) **METHOD FOR EVALUATING THE SMOOTHNESS OF A TOPSHEET OR A BACKSHEET OF A DISPOSABLE ABSORBENT ARTICLE**

(71) Applicant: The Procter & Gamble Company, Cincinnati, OH 45202 (US)
(72) Inventor: WIESEMANN, Frank, 65824 Schwalbach am Taunus (DE); SANBORN, Sarah Ann, Cincinnati, OH Ohio 45202 (US); HOOVER, Joann, Cincinnati, OH Ohio 45202 (US)
(74) Representative: L'Huillier, Florent Charles

(57) **Abstract**

A method of evaluating the smoothness of a topsheet or a backsheet of a disposable absorbent article is disclosed, the method comprising:
- providing an element having a surface,
- providing a first disposable absorbent article having a topsheet, a backsheet, and an absorbent core located between the topsheet and the backsheet,
- rubbing the surface of the element against the topsheet or the backsheet of the disposable absorbent article along a predetermined length of the disposable absorbent article with a predetermined force,
- inspecting the surface of the element after it has been rubbed to evaluate if the surface of the element or the topsheet or the backsheet of the disposable absorbent article is damaged or destroyed by the rubbing against the topsheet or the backsheet of the disposable absorbent article.

## Description

### FIELD OF THE INVENTION

The invention concerns a method for assessing characteristics of disposable absorbent articles and more particularly in assessing characteristics of topsheets or backsheets of disposable absorbent articles. The present invention concerns particularly a method for evaluating the smoothness of an absorbent article, preferably a disposable absorbent article, such as a diaper.

### BACKGROUND OF THE INVENTION

Absorbent articles, such as diapers and adult incontinence products are well known. Typically, such articles comprise a liquid pervious topsheet that faces the wearer's body, a liquid impervious backsheet that faces the wearer's clothing and an absorbent core interposed between the topsheet and the backsheet.

Since being introduced to the market place, disposable absorbent articles continue to improve regarding comfort, fit and functionalities. Topsheets of such disposable absorbent articles may function to absorb and/or contain the discharged materials and also to isolate bodily exhudates from the wearer's skin and from the wearer's garments and bed clothing. Backsheets may function to contain the discharged materials to avoid any leakage and may be smooth. While absorption capacity is quite important for diapers, consumers also appreciate products having a smooth feel to the touch, especially in regions of the article which directly contact the skin of the wearer, such as a diaper topsheet. In some instances, some topsheets may be perceived as being uneven and rough. Also a lack of smoothness may compromise the skin of the wearer. Due to contact with clothes, backsheets which do not show sufficient smoothness may exhibit fuzz and pilling. Attempts have been made to increase the smoothness of the topsheet, such as by selecting a particular fiber type or by reducing fibers bonding within the material. Assesment of the smoothness of a disposable absorbent article can be done by touching the disposable absorbent article, thus evaluating the degree of smoothness of the article. However, this method is rather subjective and difficult to reproduce. In addition, the smoothness of a disposable absorbent article cannot be effectively visually demonstrated. As such, there is a need for improved method for evaluating the smoothness of a disposable absorbent article that exhibits good reproducibility and that is well visually demonstrated.

The object of the present invention relates to a method of evaluating the smoothness of a topsheet or a backsheet of a disposable absorbent article, the method comprising the steps of: providing an element having a surface, providing a disposable absorbent article having a topsheet, a backsheet, and an absorbent core located between the topsheet and the backsheet, rubbing the surface of the element against the topsheet or the backsheet of the disposable absorbent article along a predetermined length of the disposable absorbent article with a predetermined force, inspecting visually the surface of the element after it has been rubbed to evaluate if the surface of the element or the surface of the topsheet or the backsheet of the disposable absorbent article is damaged or destroyed by the rubbing against the topsheet or the backsheet of the disposable absorbent article.

The element maybe selected from the group consisting of: a petal, a plant leaf, a coloring tool such as chalks or a fruit such as peach.

The element and the disposable absorbent article may be visually distinguished, such as by the naked eye, due to a difference in color.

The predetermined length may be between 30 mm and 400 mm, particularly between 100 mm and 350 mm.

The surface of the element may be rubbed on more than 10% of the length of the disposable absorbent article on the longitudinal axis, particularly more than 50% of the length of the disposable absorbent article, and not more than 90% of the length of the disposable absorbent article.

The step of rubbing the surface of the element against the topsheet or the backsheet of the disposable absorbent article maybe repeated at least two times, particularly three times.

The predetermined force maybe less than 6 N, particularly less than 5 N.

The element chosen, the predetermined length, the predetermined force, the repeated time the surface of the element is rubbed against the topsheet or the backsheet of the disposable absorbent article and the topsheet or the backsheet used determine if the surface of the element will be damaged or destroyed.

The disposable absorbent article may a first disposable absorbent article and the element may be a first element.

The smoothness of the first disposable absorbent article and the smoothness of a second disposable absorbent article maybe evaluated and compared.

The second disposable absorbent article is different from the first disposable absorbent article at least with respect to the topsheet if the smoothness of the topsheet is evaluated or with respect to the backsheet if the smoothness of the backsheet is evaluated.

In addition to evaluating the smoothness of the first absorbent article, the smoothness of a second disposable absorbent article may be evaluated by a method comprising the step of: providing a second element having a surface, providing a second disposable absorbent article having a topsheet, a backsheet, and an absorbent core located between the topsheet and the backsheet, rubbing the surface of the second element against the topsheet or backsheet of the second disposable absorbent article along a predetermined length of the second disposable absorbent article with a predetermined force, inspecting the surface of the second element after it has been rubbed to evaluate if the surface of the second element or the topsheet or the backsheet of the second disposable absorbent article is damaged or destroyed by the rubbing against the topsheet or backsheet of the second disposable absorbent article.

According to the invention, the first element and the second element, the predetermined length and the predetermined force applied on the first disposable absorbent article and on the second disposable absorbent article are the same.

The step of rubbing the surface of the second element against the topsheet or the backsheet of the second disposable absorbent article may be repeated at least two times, particularly three times.

The number of repeated time the surface of the first element and the surface of the second element is rubbed against the topsheet or the backsheet of the first disposable absorbent article and of the second disposable absorbent article may be the same.

The surface of the second element or the topsheet or the backsheet of the second disposable absorbent article may be damaged or destroyed when the surface of the second element is rubbed against the topsheet or backsheet of the second disposable absorbent article but the surface of the first element or the topsheet or the backsheet of the first disposable absorbent article may be not damaged or destroyed when the surface of the first element is rubbed against the topsheet or backsheet of the first disposable absorbent article.

This method is used to compare the smoothness of two types of disposable absorbent article that do not comprise the same topsheet if the smoothness of the topsheet is evaluated or do not comprise the same backsheet if the smoothness of the backsheet is evaluated.

This method allows to demonstrate which disposable absorbent article presents the best smoothness; that is to say which topsheet or backsheet of a disposable absorbent article presents the best smoothness.

This method for evaluating the smoothness of a disposable absorbent article exhibits good reproducibility and is well visually demonstrated.

### BRIEF DESCRIPTION OF THE DRAWINGS

Figure 1 is a plan view of a disposable absorbent article, more particularly a diaper.
Figure 2 shows an exemplary illustration of an element that may be used for the smoothness test.
Figure 3A shows a plan view of a disposable absorbent article and an element before the element is rubbed against the topsheet of this disposable absorbent article.
Figure 3B shows a plan view of a disposable absorbent article and an element after the element is rubbed against the topsheet of the disposable absorbent article and shows that the element is not damaged by rubbing it against the topsheet of the disposable absorbent article.
Figure 3C shows a plan view of a disposable absorbent article and an element after the element is rubbed against the topsheet of the disposable absorbent article and shows that the element is damaged by rubbing it against the topsheet of the disposable absorbent article.
Figure 4A is a plan view of the destroyed element.
Figure 4B is a plan view of the damaged element of Figure 3C.
Figure 4C shows an exemplary illustration of a peach with damage on it surface caused by rubbing the peach against the topsheet of the disposable absorbent article.
Figure 5A is a plan view of a first disposable absorbent article such as a first diaper and a second disposable absorbent article such as a second diaper having differents topsheets and of a first element and a second element that is going to be rubbed against the topsheet of each diaper.
Figure 5B is a plan view of a first disposable absorbent article such as a first diaper and a second disposable absorbent article such as a second diaper having differents topsheets and of a first element and a second element after they have been rubbed against the topsheet of each diaper, illustrating that, on one hand, the first element is not damaged by rubbing it against the topsheet of the first diaper and, on the other hand, the second element is damaged by rubbing it against the topsheet of the second diaper.
Figure 6 is a profile view of a first disposable absorbent article that is curved.

### DETAILED DESCRIPTION OF THE INVENTION

The following term explanations my be useful in understanding the present disclosure:
"Absorbent article" refers to devices that absorb and contain body exudates, and, more specifically, refers to devices that are placed against or in proximity to the body of the wearer to absorb and contain the various exudates discharged from the body. Absorbent articles may include diapers (baby diapers and diapers for adult incontinence), pants, inserts, feminine care absorbent articles such as sanitary napkins or pantiliners, and the like. As used herein, the term "exudates" includes, but is not limited to, urine, blood, vaginal discharges, sweat and fecal matter. Preferred absorbent articles of the present invention are disposable absorbent articles, more preferably disposable diapers and disposable pants.
"Disposable" is used in its ordinary sense to mean an article that is disposed or discarded after a limited number of usage over varying lengths of time, for example, less than 20 usages, less than 10 usages, less than 5 usages, or less than 2 usages. If the disposable absorbent article is a diaper, a pant, sanitary napkin, sanitary pad or wet wipe for personal hygiene use, the disposable absorbent article is most often intended to be disposed after single use.
"Diaper" and "pant" refers to an absorbent article generally worn by babies, infants and incontinent persons about the lower torso so as to encircle the waist and legs of the wearer and that is specifically adapted to receive and contain urinary and fecal waste. In a pant, as used herein, the longitudinal edges of the first and second waist region are attached to each other to a pre-form waist opening and leg openings. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (e.g., seam, weld, adhesive, cohesive bond, fastener, etc.). A pant may be preformed anywhere along the circumference of the article (e.g., side fastened, front waist fastened). In a diaper, the waist opening and leg openings are only formed when the diaper is applied onto a wearer by (releasably) attaching the longitudinal edges of the first and second waist region to each other on both sides by a suitable fastening system.
A "pantiliner" and a "sanitary napkin" generally have two end regions and a middle region (i.e. a crotch region). The pantiliner and the sanitary napkin has a body-facing surface and a garment facing surface. The size and shape of the absorbent structure positioned between the topsheet and the backsheet can be altered to meet absorbent capacity requirements, and to provide comfort to the wearer. The garment facing surface of the pantiliner and of the sanitary napkin can have thereon pressure sensitive adhesive for affixing to a wearer's undergarments. Typically, such adhesive is covered with a release strip which is removed before affixing to the undergarment. Pantiliners can also be provided with lateral extensions known commonly in the art as "flaps" or "wings" intended to extend over and cover the panty elastics in the crotch region of the user's undergarment. However, wings are normally not used with pantiliners but are more often used in sanitary napkins. Sanitary napkins and pantiliners of the present invention may comprise barrier leg cuffs.
"Rubbing"refers to the effort expended in moving one object over and against another with a certain force. There is a resistance encountered when one body is moved in contact with another.
"Damage" means a physical harm that impairs the value, the usefulness or the normal function of an object, such as the element described below. "Destroy" refers to a more severe damage, that is to say an action that reduce an object to useless fragments.
"Comprise", "comprising, and "comprises" is an open ended term that specifies the presence of what follows e.g., a component but does not preclude the presence of other features, elements, steps or components known in the art, or disclosed herein. The term includes the terms "consist", "consisting".
"Longitudinal" means a direction running substantially perpendicular from a waist edge (or end edge) to a longitudinally opposing waist edge (or opposing end edge) of an absorbent article when the article is in a flattened out, uncontracted state. The distance between an end edge edge to the opposing end edge is the length of the article. Directions within 45 degrees of the longitudinal direction are considered to be "longitudinal." "Lateral" refers to a direction running from a longitudinally extending side edge to a laterally opposing longitudinally extending side edge of an article and generally at a right angle to the longitudinal direction. Directions within 45 degrees of the lateral direction are considered to be "lateral."
A "nonwoven web" is a manufactured web of directionally or randomly oriented fibers, consolidated and bonded together. The term does not include fabrics which are woven, knitted, or stitch-bonded with yarns or filaments. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as meltblowing, spunbonding, solvent spinning, electrospinning, and carding. Nonwoven webs may be bonded by heat and/or pressure or may be adhesively bonded. Bonding may be limited to certain areas of the nonwoven web (point bonding, pattern bonding). Nonwoven webs may also be hydro-entangled or needle-punched. The basis weight of nonwoven fabrics is usually expressed in grams per square meter (g/m²).

### Evaluating the smoothness of a disposable absorbent article

The present invention relates to a method for evaluating the smoothness of an absorbent article, preferably a disposable absorbent article, such as a diaper. Although embodiments described in detail below may describe a diaper, the claimed methods and apparatuses may be used to evaluate the smoothness of any other absorbent articles.

In this application, the method concerns the evaluation of the smoothness of the topsheet or the backsheet of a disposable absorbent article. Evaluation is done on assembled absorbent articles (i.e not on the topsheet or the backsheet in isolation). For example, for a diaper, the smoothness of the backsheet, the absorbent core and the topsheet is evaluated on the assembled product in order to reflect in-use condition wherein the smoothness of the topsheet or the backsheet maybe impacted by underlying layers.

While the following description relates to the evaluation of the smoothness of the topsheet of a disposable absorbent article, the description is equally applicable for the assessment of the backsheet smoothness of a disposable absorbent article.

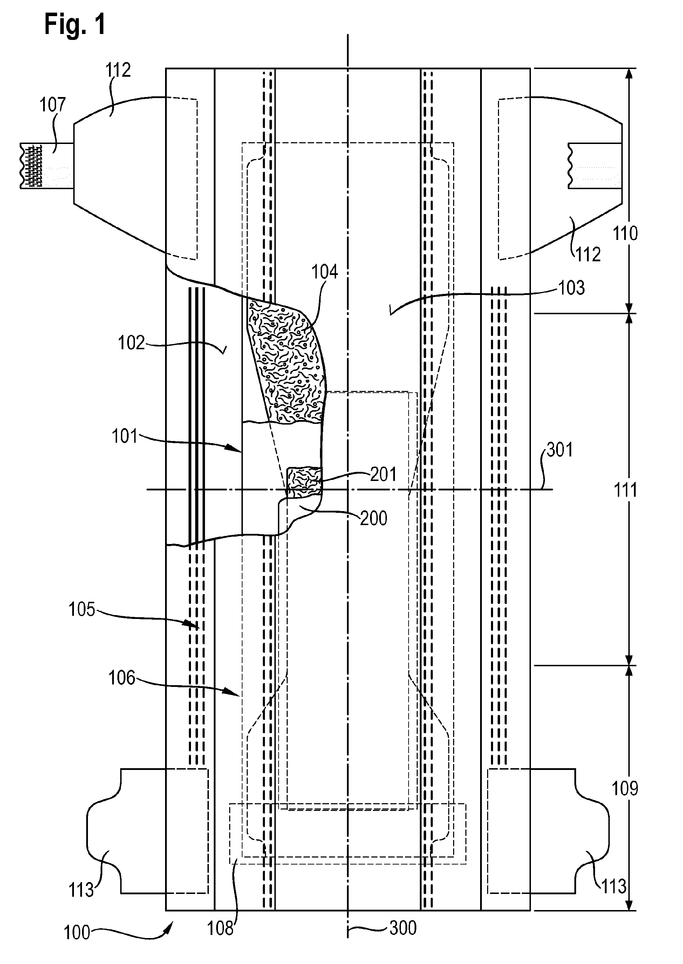

A typical absorbent article is represented in Fig. 1 in the form of a diaper 100. However, the structure may be applied in other disposable absorbent articles, such as pants, pantiliners and sanitary napkins in the same manner and configuration as described herein below for a diaper.

In more details, Figure 1 is a plan view of an exemplary diaper 100, in a flattened out state, with portions of the diaper being cut-away to more clearly show the construction of the diaper 100. As said, this diaper 100 is shown for illustration purpose only as the structure of the present invention may be comprised in a wide variety of diapers or other absorbent articles.

As shown in Figure 1, the absorbent article, here a diaper, comprises a liquid pervious topsheet 103, a liquid impervious backsheet 102, an absorbent core 101 which is preferably positioned between at least a portion of the topsheet 103 and the backsheet 102. The absorbent core 101 can absorb and contain liquid received by the absorbent article and may comprise absorbent materials 104, such as superabsorbent polymers and/or cellulose fibers, as well as other absorbent and non-absorbent materials commonly used in absorbent articles (e.g. thermoplastic adhesives immobilizing the superabsorbent polymer particles). The diaper 100 may also include optionally an acquisition system with an upper acquisition layer 200 and a lower acquisition layer 201.

The diaper may also comprise barrier leg cuffs 106 and may further comprise elasticized leg cuffs 105. Moreover, the absorbent article may comprise a fastening system, such as an adhesive fastening system or a hook and loop fastening system, which can comprise tape tabs 107, such as adhesive tape tabs or tape tabs comprising hook elements, cooperating with a landing zone 108 (e.g. a nonwoven web providing loops in a hook and loop fastening system). The diaper 100 as shown in Figure 1 can be notionally divided in a first waist region 109, a second waist region 110 opposed to the first waist region 109 and a crotch region 111 located between the first waist region 109 and the second waist region 110. The longitudinal axis 300 is the imaginary line separating the diaper along its length in two equal halves. The transversal axis 301 is the imagery line perpendicular to the longitudinal axis 300 in the plane of the flattened out diaper and going through the middle of the length of the diaper. The periphery of the diaper 100 is defined by the outer edges of the diaper 100. The longitudinal edges of the diaper may run generally parallel to the longitudinal axis 300 of the diaper 100 and the end edges run between the longitudinal edges generally parallel to the transversal axis 301 of the diaper 100. The crotch region, the first and the second waist region each constitutes 1/3 of the absorbent article along the longitudinal axis

Further, the diaper may comprise other elements, such as a back waist feature, which may be non-elastic or elastic, and a front waist feature, which may be non-elastic or elastic, a lotion applied onto the wearer-facing surface of the topsheet, back ears 112, and/or front ears 113. The front and/or back ears 112, 113 may be separate components attached to the diaper or may instead be continuous with portions of the topsheet and/or backsheet and/or even portions of the absorbent core such that these portions form all or a part of the front and/or back ears 112, 113. Also combinations of the aforementioned are possible, such that the front and/or back ears 112, 113 are formed by portions of the topsheet and/or backsheet while additional materials are attached to form the overall front and/or back ears 112, 113. The front and/or back ears may be elastic or non-elastic.

The topsheet 103, the backsheet 102, and the absorbent core 101 may be assembled in a variety of well known configurations, in particular by gluing or heat embossing. Exemplary diaper configurations are described generally in US3,860,003; US5,221,274; US5,554,145; US5,569,234; US5,580,411; and US6,004,306.

The topsheet 103 and the backsheet 102 maybe nonwoven webs.

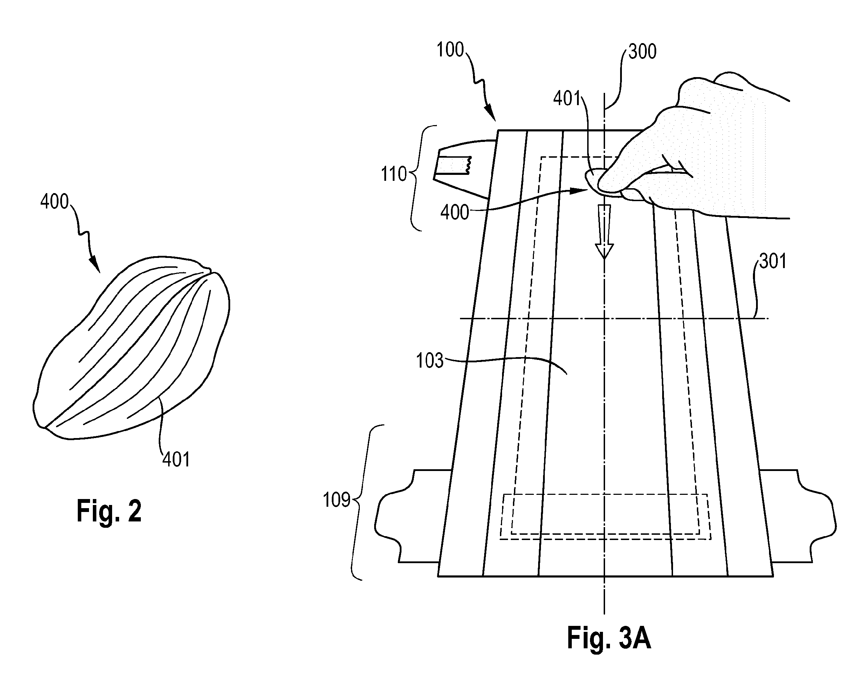

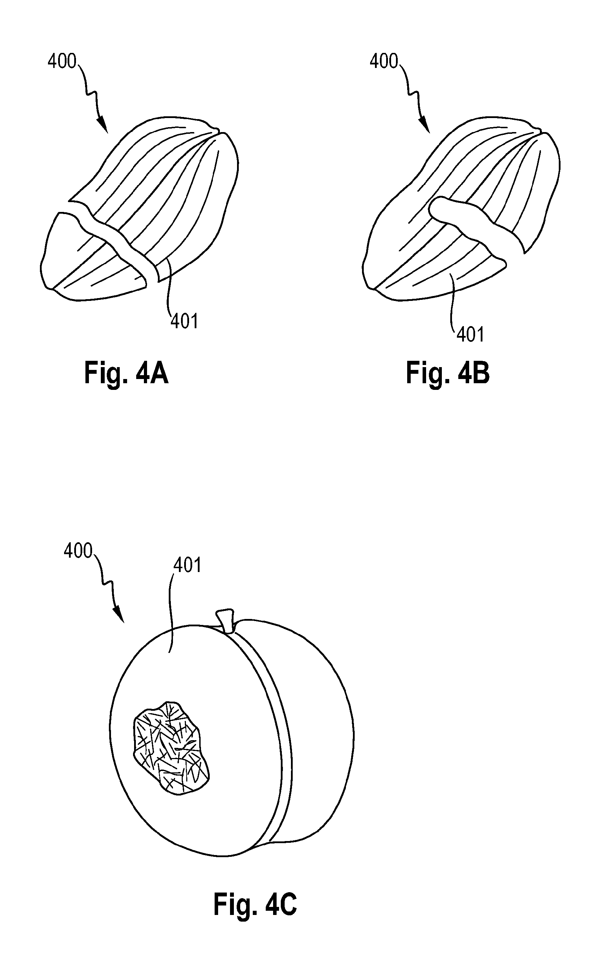

Figure 2 is an illustration of an element 400 that may be used to assess the smoothness of a disposable absorbent article, such as a diaper. The method may involve the partial to total destruction of the surface 401 of the element 400, such as shown in Figure 4A, Figure 4B and Figure 4C.

The element 400 may be any sensitive article that may be damaged or destroyed by rubbing it against the topsheet of a disposable absorbent article.

The element 400 may be selected from the group consisting of: a petal, a plant leaf such as aloe leaf, a coloring tool such as chalks or a fruit such as peach. For example, the petal can be selected from the group consisting of orchid petal, red rose petal, white rose petal, peach rose petal, yellow rose petal and petunia petal. Particularly, the element is a petal, more particularly the element is an orchid petal.

The element 400 is represented in Figures 2, 3A-C, 4A-B and 5A-B in the form of an orchid petal. The orchid petal is shown for illustration purpose only as the present invention may imply a wide variety of element, such as shown in Figure 4C with the element in the form of a peach.

The element and the disposable absorbent article may be visually distinguished from each other, such as by the naked eye, due to a difference in color.

The difference of color between the two items allow to clearly distinguish the element and the disposable absorbent article. The fuzz of the disposable absorbent article on the element can be visible. For example, if the smoothness of the topsheet of the disposable absorbent article is evaluated, fibers of the nonwoven topsheet may be visible on the surface of the element. If the smoothness of the backsheet of the disposable absorbent article is evaluated, fibers of the nonwoven backsheet may be visible on the surface of the element.

Moreover, pieces of the broken element can also be visible on the topsheet or the backsheet of the disposable absorbent article.

Figure 3A illustrate the smoothness test according to the invention.

The element 400, for example an orchid petal, is inspected before the test to ensure that it does not present any damage on this surface 401.

The disposable absorbent article such as a diaper 100 maybe flat or curved. Particularly, the disposable absorbent article is curved.

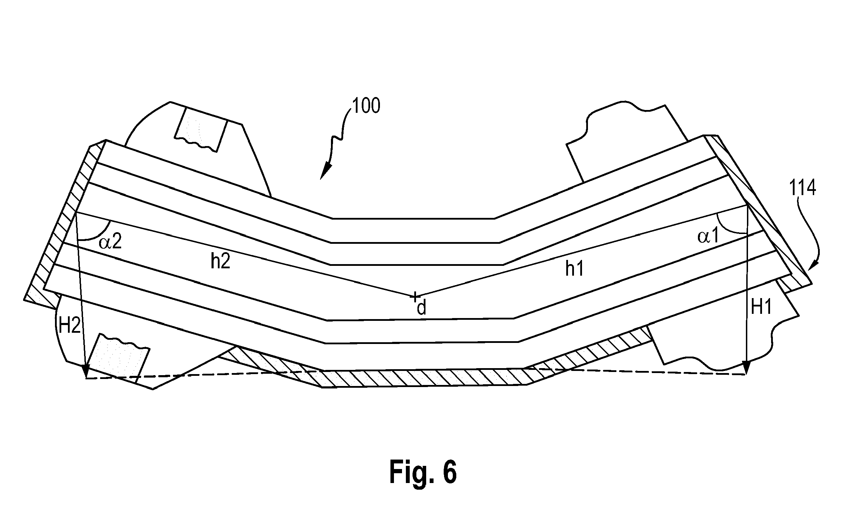

Figure 6 is an illustration of a curved diaper 100. The distance between the waist edge of the first waist region of the diaper 100 and the middle point d of the crotch region is the distance h₁. The distance h₁ is between 5 cm to 50 cm. The distance between the waist edge of the first waist region of the diaper 100 and the crotch portion is the distance H₁. The distance H₁ is between 0 cm and 10 cm. The intersection between h₁ and H₁ defines an angle α₁. The diaper 100 may be curved from an angle α₁ comprised between 0° and 45°, particularly between 5° and 25°. The angle α₁ is less than 45°, particularly less than 25°.

The distance between the waist edge of the second waist region of the diaper 100 and the middle point d of the crotch region is the distance h₂. The distance h₂ is between 5 cm to 50 cm. The distance between the waist edge of the first waist region of the diaper 100 and the crotch portion is the distance H₂. The distance H₂ is between 0 cm and 10 cm. The intersection between h₂ and H₂ defines an angle α₂. The diaper 100 may be curved from an angle α₂ comprised between 0° and 45°, particularly between 5° and 25°. The angle α₂ is less than 45°, particularly less than 25°.

The advantage of having a flat or curved diaper with an angle α₁ up to 45° and an angle α₂ up to 45° is to avoid the emergence of wrinkles on the topsheet of the diaper.

A curved rig 114 may be used to curve the diaper.

The disposable absorbent article, for example a diaper 100, maybe dry.

As shown in Figure 3A, the surface 401 of the element 400, such as an orchid petal, is rubbed against the topsheet 103 of the diaper 100 along a predetermined length of the diaper 100 with a predetermined force.

In order to rub the surface 401 of the element 400 against the topsheet 103 of the diaper 100, the surface 401 of the element 400 may be held by a hand, for example, the hand of the person making the test or by any other object, for example a mechanical arm.

The surface 401 of the element 400 maybe rubbed along the longitudinal axis 300 of the diaper 100 from the first waist region 109 towards the second waist region 110. Alternatively, the surface 401 of the element 400 may be rubbed along the longitudinal axis 300 of the diaper 100 from the second waist region 110 towards the first waist region 109. In another alternative, the surface 401 of the element 400 may be rubbed along other directions such as along the lateral axis 301 of the diaper 100 or the radials axis of the diaper 100.The predetermined length along which the surface 401 of the element 400 is rubbed against the topsheet 103 of the diaper 100 may be between 30 mm and 400 mm, particularly between 100 mm and 350 mm.

The surface of the element may be rubbed over more than 10% of the length of the topsheet 103 of the diaper 100 on the longitudinal axis 300, particularly more than 50% of the length of the topsheet 103 of the diaper 100, and not more than 90% of the length of the diaper 100.

The predetermined force with which the surface 401 of the element 400 is rubbed against the topsheet 103 of the diaper 100 maybe less than 6 N, particularly less than 5 N.

An appropriate predetermined force inter alia depends on the element to be rubbed against the topsheet or the backsheet of the disposable absorbent article. Elements having a rather fragile, delicate surface may be rubbed with a lower force compared to elements having a somewhat more robust surface.

The surface 401 of the element 400 may be rubbed against the topsheet 103 of the diaper 100 at least two times, particularly three times.

The surface 401 of the element 400 is inspected visually after it has been rubbed to evaluate if the surface of the element 400 or the topsheet 103 of the diaper 100 is damaged or destroyed by the rubbing against the topsheet 103 of the diaper 100.

The surface of the element or the topsheet or the backsheet of the disposable absorbent article is inspected visually, that is to say by the naked eyes, or by using a microscope, or with magnifying glass or other similar object.

Fibers of the nonwoven topsheet 103 of the diaper 100 maybe visible on the surface 401 of the first element 400. Pieces of the element 400 may also be visible on the topsheet 103 of the first diaper 100 .

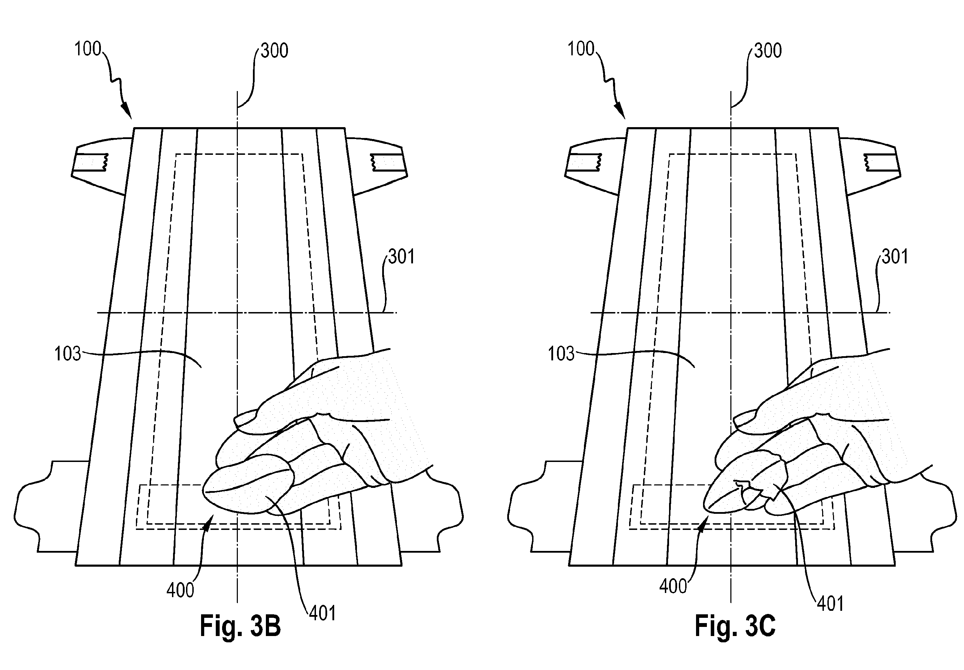

As shown in Figure 3B, the surface 401 of the element 400 is not damaged or destroyed. As shown in Figure 3C, the surface 401 of the element 400 is damaged or destroyed.

The result allows the person making the test to have some qualitative data relating to smoothness.

Figures 4A and 4B are plan view of an exemplary element 400, such as an orchid petal, that is damaged or destroyed after the implementation of the test. The destruction of the element 400 results in the break of the element 400 into several pieces as shown in Figure 4A. The damage of the element 400 results in the tear of the surface 401 of the element 400 as shown in Figure 4B.

Figure 4C shows an exemplary illustration of a peach that maybe used as an element 400 in order to be rubbed against the topsheet 103 of the diaper 100 in order to evaluate the smoothness of the diaper. The element 400 of Figure 4C has an outer layer having a fluid under the outer layer and damage to the element results in the fluid becoming visible, or leaking upon damage to the element.

The element 400 may have several adjacent layers. Adjacent layers may have different properties, such as, for example, different colors or textures. As one layer of the element 400 is damaged or destroyed, the underlying adjacent layer may become visible and provide a visualization of the degree of damage. Similarly, adjacent layers may contain liquid with different properties, such as for example, a yellow liquid may be contained between the first and second layers, and a blue liquid may be contained between the second and the third layers. Accordingly, as each layer of the element 400 is damaged or destroyed, the liquid contained between succeeding adjacent layers may escape, and thus the degree of damage becomes visible by the color of fluid that is escaping from the element 400.

### Evaluating and comparing the smoothness of a first disposable absorbent article with a second disposable absorbent article

The disposable absorbent article described above may be a first disposable absorbent article and the element described above maybe a first element.

The description provided is also applicable for the evaluation of the smoothness of a second disposable absorbent article and for the comparison of the smoothness of a first disposable absorbent article with a second disposable absorbent article.

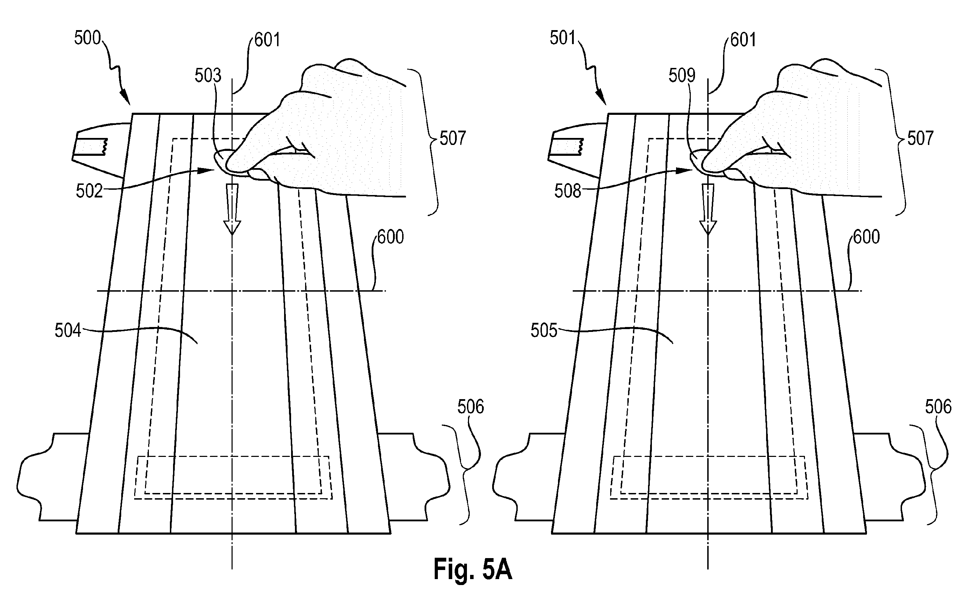

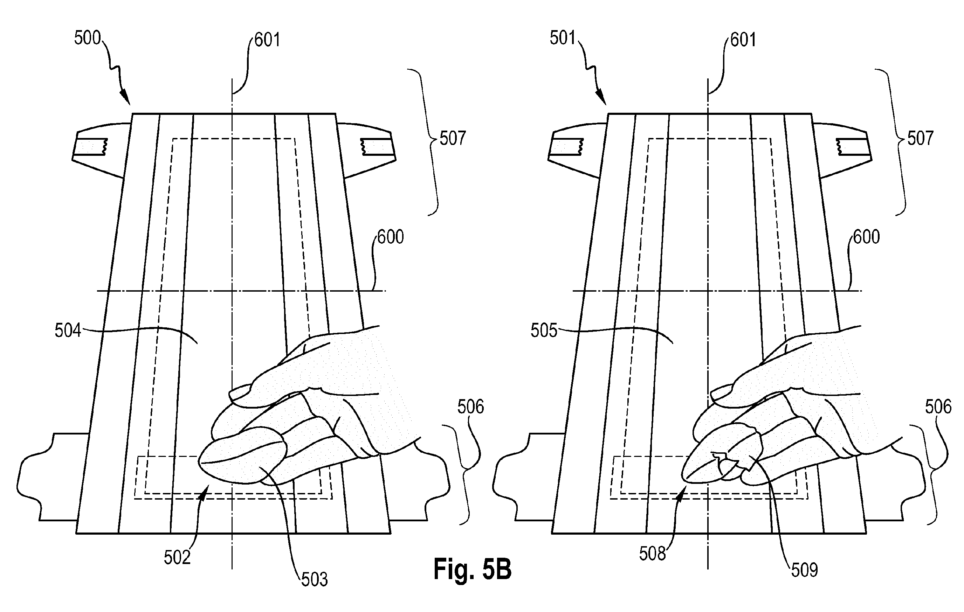

Figures 5A and 5B illustrate the smoothness test according to the invention comparing the smoothness of a first disposable absorbent article 500 to a second disposable absorbent article 501, using a first element 502 and a second element 508, such as orchid petals. The first disposable absorbent article 500 and the second disposable absorbent article 501 are here diapers which have different topsheets. When comparing the smoothness of the first diaper 500 and the second diaper 501, each of the diapers are tested with the first element 502 and the second element 508 in substantially the same manner. One method of ensuring substantially the same manner is first to determine a predetermined length along which the surface 503 of the first element 502 is rubbed against the topsheet 504 of the first diaper 500 and the surface 509 of the second element 508 is rubbed against the topsheet 505 of the second diaper 501. Second, a predetermined force applied on the topsheet 504 of the first diaper 500 and on the topsheet 505 of the second diaper 501 is also determined to ensure the same procedure during the test. In addition, using similar elements helps to avoid variations. The first element 502 and the second element 508 have substantially the same surface properties. For example, two similar petals of the same orchid may be used that is to say that the first element may be an orchid petal and the second element may be an orchid petal of the same blossom.

If the smoothness of the topsheet is evaluated, the topsheet 504 of the first diaper 500 is different from the topsheet 505 of the second diaper 501.

The topsheet of the first diaper 500 and of the second diaper 501 may not have lotion. In contrary, the topsheet of the first diaper 500 and of the second diaper 501 may have lotion.

If the smoothness of the backsheet is evaluated, the backsheet of the first diaper 500 is different from the backsheet of the second diaper 501.

According to the invention, the first element 502, the second element 508, the predetermined length and the predetermined force applied on the first diaper 500 and on the second diaper 501 are the same.

As shown in Figure 5A, after inspecting visually the surface 503 of the first element 502, such as an orchid petal, to ensure that it does not present any damage on this surface 503, the surface 503 of the first element 502 is rubbed against the topsheet 504 of the first diaper 500 along a predetermined length of the first diaper 500 with a predetermined force.

The surface 503 of the first element 502 may be rubbed along the longitudinal axis 601 of the first diaper 500 from the first waist region 506 towards the second waist region 507. Alternatively, the surface 503 of the first element 502 may be rubbed along the longitudinal axis 601 of the first diaper 500 from the second waist region 507 towards the first waist region 506.

In another alternative, the surface 503 of the first element 502 maybe rubbed along other directions such as along the lateral axis 600 of the first diaper 500 or the radials axis of the first diaper 500.

In parallel, as shown in Figure 5A, after inspecting visually the surface 509 of the second element 508, such as an orchid petal from the same blossom, to ensure that it does not present any damage on this surface 509, the surface 509 of the second element 508 is rubbed against the topsheet 505 of the second diaper 501 along the same predetermined length of the first diaper 500 with the same predetermined force.

The surface 509 of the second element 508 maybe rubbed along the longitudinal axis 601 of the second diaper 501 from the first waist region 506 towards the second waist region 507. Alternatively, the surface 509 of the second element 508 may be rubbed along the longitudinal axis 601 of the second diaper 501 from the second waist region 507 towards the first waist region 506. In another alternative, the surface 509 of the second element 508 maybe rubbed along other directions such as along the lateral axis 600 of the second diaper 501 or the radials axis of the second diaper 501. The surface 503 of the first element 502 and the surface 509 of the second element 508 maybe rubbed on the same direction on the topsheet 504 of the first diaper 500 and on the topsheet 505 of the second diaper 501.

The predetermined length and the predetermined force are the same as indicated above.

The surface 503 of the first element 502 and the surface 509 of the second element 508 may be rubbed against the topsheet 504 of the first diaper 500 and the topsheet 505 of the second diaper 501 at least two times, particularly three times. The number of repeated time the surface 503 of the first element 502 and the surface 509 of the second element 508 is rubbed against the topsheet 504 of the first diaper 500 and against the topsheet 505 of the second diaper 501 may be the same.

As shown in Figure 5B, the surface 503 of the first element 502, for example, an orchid petal, is inspected visually after it has been rubbed against the first diaper 500 to evaluate if the surface of the first element or the topsheet 504 of the first diaper 500 is damaged or destroyed by the rubbing against the topsheet 504 of the first diaper 500.

Fibers of the nonwoven topsheet 504 of the first diaper 500 maybe visible on the surface 503 of the first element 502. Pieces of the first element 502 may also be visible on the topsheet 504 of the first diaper 500 .

In parallel, the surface 509 of the second element 508, for example, an orchid petal of the same blossom is inspected after it has been rubbed against the second diaper 501 to evaluate if the surface 509 of the second element 508 or the topsheet 505 of the second diaper 501 is damaged or destroyed by the rubbing against the topsheet 505 of the second diaper 501.

Fibers of the nonwoven topsheet 505 of the second diaper 501 may be visible on the surface 509 of the second element 508. Pieces of the second element 508 may also be visible on the topsheet 505 of the second diaper 501.

The surface 503 of the element 502 and the surface 509 of the second element 508 may be inspected visually or by using a microscope or magnifying glass or other similar object.

As shown on Figure 5A and Figure 5B, the surface 509 of the second element 508 is damaged or destroyed when it is rubbed against the topsheet 505 of the second diaper 501 but the surface 503 of the first element 502 is not damaged or destroyed when it is rubbed against the topsheet 504 of the first diaper 500.

The results from the smoothness test demonstrate that the first diaper 500 is smoother than the second diaper 501.

This method of demonstrating the smoothness of a disposable absorbent article provides a repeatable visualization of the smoothness of a diaper. This visualization may be readily communicated to a consumer who can visually see the benefits of using the smoother diaper. The visualization may be, for example, a qualitative measurement, a display for consumer education, a close-up of the destructible article, a histogram, a line chart, a side-by-side comparison, a tabulation of data, video images, displays, and print adds. Visualizations could also be shown to consumers in the store as part of a display, or other promotional materials.

### Method to measure the force applied on the topsheet or the backsheet of a disposable

### absorbent article:

A disposable absorbent article, such as a diaper is applied to a rigid Lexan plate with the topsheet or the backsheet oriented upwards in a flat or curved configuration.

The Lexan plate is attached to a balance connected to a computer.

An orchid petal is taken from an orchid plant and is used for the test.

The orchid petal is rubbed against the topsheet or the backsheet of the disposable absorbent article in the machine direction (MD) while the force was measured by the balance and recorded by the computer at interval of 0.1 s. The orchid petal is rubbed against the topsheet or the backsheet of the disposable absorbent article at a speed of 60 to 70 mm/s. The predetermined force that is measured is the downwards force.

### Example:

A smoothness test was implemented to evaluate the smoothness of a Premium Care Pampers® diaper and to compare the smoothness of the Premium Care Pampers® diaper with the smoothness of a Merries® diaper.

The test consisted of evaluating the smoothness of a topsheet of the Premium Care Pampers® diaper with the smoothness of a topsheet of a Merries® diaper.

### The material used:

Premium Care Pampers® diaper used was a diaper size L sold in China in January 2015. The topsheet of this commercially available diaper was replaced with a nonwoven topsheet made of PE/PP core/sheath bicomponent fibers with a weight of 25gsm (PE sheath and PP core with a ratio of 50:50). The topsheet had a substantially flat surface.

Merries® diaper used was a diaper size L, purchased in China in January 2015. The topsheet of the Merries® diaper was an nonwoven topsheet with a three dimensional surface.

Two similar orchid petals of the same blossom were used.

### Protocol:

First of all, a first orchid petal was rubbed against the topsheet of the Pampers® diaper along the longitudinal axis of the diaper from the second waist region to the first waist region along a length of 330 mm.

In parallel, a second orchid petal from the same blossom was rubbed against the topsheet of the Merries® diaper along the longitudinal axis of the diaper from the second waist region to the first waist region along a length of 330 mm.

The movement of rubbing the orchid petal against the topsheet of each diaper was repeated three times.

### Result:

The surface of the two orchid petals was visually inspected to evaluate if the surface of the orchid petal is damaged or destroyed.

The second orchid petal that was rubbed against the topsheet of the Merries® diaper was damaged. Indeed, the damage of the orchid petal resulted in the tear of the orchid petal.

However, the first orchid petal that was rubbed against the topsheet of the Pampers® diaper was not damaged. Indeed, the surface of the orchid petal did not show any tear or rip.

The result indicated that the Pampers® diaper presented a topsheet that was smoother than the topsheet of the Merries® diaper.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A method of evaluating the smoothness of a topsheet or a backsheet of a disposable absorbent article, the method comprising the steps of:
- providing an element having a surface,
- providing a disposable absorbent article having a topsheet, a backsheet, and an absorbent core located between the topsheet and the backsheet,
- rubbing the surface of the element against the topsheet or the backsheet of the disposable absorbent article along a predetermined length of the disposable absorbent article with a predetermined force,
- inspecting visually the surface of the element after it has been rubbed to evaluate if the surface of the element or the topsheet or the backsheet of the disposable absorbent article is damaged or destroyed by the rubbing against the topsheet or the backsheet of the disposable absorbent article.

2. The method of claim 1 wherein the element is selected from the group consisting of: a petal, a plant leave, a coloring tool such as chalks or a fruit such as peach.

3. The method according to any of the preceding claims, wherein the element and the disposable absorbent article are visually distinguished, such as by the naked eye, due to a difference in color.

4. The method according to any of the preceding claims, wherein the topsheet and the backsheet are nonwoven webs.

5. The method according to any of the preceding claims, wherein the predetermined length is between 30 mm and 400 mm, particularly between 100 mm and 350 mm.

6. The method according to any of the preceding claims, wherein the disposable absorbent article has a longitudinal axis and a lateral axis.

7. The method of claim 6, wherein the surface of the element is rubbed on more than 10% of the length of the disposable absorbent article on the longitudinal axis, particularly more than 50% of the length of the disposable absorbent article and not more than 90% of the length of the disposable absorbent article.

8. The method according to any of the preceding claims, wherein the step of rubbing the surface of the element against the topsheet or the backsheet of the disposable absorbent article is repeated at least two times, particularly three times.

9. The method according to any of the preceding claims, wherein the predetermined force is less than 6 N, particularly less than 5 N.

10. The method according to any of the preceding claims, wherein the disposable absorbent article is dry.

11. The method according to any of the preceding claims, wherein the disposable absorbent article is flat or curved.

12. The method according to any of the preceding claims , wherein the disposable absorbent article is a first disposable absorbent article and the element is a first element, wherein the smoothness of the first disposable absorbent article and the smoothness of a second disposable absorbent article are evaluated and compared, wherein the second disposable absorbent article is different from the first disposable absorbent article at least with respect to the topsheet if the smoothness of the topsheet is evaluated or with respect to the backsheet if the smoothness of the backsheet is evaluated, wherein the smoothness of a topsheet or a backsheet of a second disposable absorbent article is evaluated by a method comprising the steps of:
- providing a second element having a surface,
- providing a second disposable absorbent article having a topsheet, a backsheet, and an absorbent core located between the topsheet and the backsheet,
- rubbing the surface of the second element against the topsheet or the backsheet of the second disposable absorbent article along a predetermined length of the second disposable absorbent article with a predetermined force,
- inspecting the surface of the second element after it has been rubbed to evaluate if the surface of the second element or the topsheet or the backsheet of the second disposable absorbent article is damaged or destroyed by the rubbing against the topsheet or the backsheet of the second disposable absorbent article,
wherein the first element, the second element, the predetermined length and the predetermined force applied on the first disposable absorbent article and on the second disposable absorbent article are the same.

13. The method of claim 12, wherein the step of rubbing the surface of the second element against the topsheet or the backsheet of the second disposable absorbent article is repeated at least two times, particularly three times.

14. The method according to any of claim 12 or 13, wherein the number of repeated time the surface of the first element and the surface of the second element are rubbed against the topsheet or the backsheet of the first disposable absorbent article and of the second disposable absorbent article respectively are the same.

15. The method according to any of claims 12 to 14, wherein the surface of the second element or the topsheet or the backsheet of the second disposable absorbent article is damaged or destroyed when the surface of the second element is rubbed against the topsheet or the backsheet of the second disposable absorbent article but the surface of the first element or the topsheet or the backsheet of the first disposable absorbent article is not damaged or destroyed when the surface of the first element is rubbed against the topsheet or the backsheet of the first disposable absorbent article.
